# EUROPEAN PATENT SPECIFICATION

(11) **EP 3 500 313 B1**
(45) Date of publication and mention of the grant of the patent: **14.07.2021**
(21) Application number: 17751426.2
(22) Date of filing: 16.08.2017
(51) Int. Cl.: A61L 15/26, C08L 77/00

(54) **WOUND DRESSING COMPRISING POLYMER FIBERS**
WUNDVERBAND MIT POLYMERFASERN
PANSEMENT POUR PLAIES COMPRENANT DES FIBRES POLYMÈRES

(30) Priority: 22.08.2016 EP 16185130
(43) Date of publication of application: 26.06.2019
(73) Proprietor: Friedrich-Alexander-Universität Erlangen-Nürnberg, 91054 Erlangen (DE); Universitätsklinikum Erlangen, 91054 Erlangen (DE)
(72) Inventor: SCHUBERT, Dirk, Wolfram, 91330 Eggolsheim (DE); FUCHSLUGER, Thomas, 91341 Röttenbach (DE); KÜNG, Florian, 91080 Uttenreuth (DE); STAFIEJ, Piotr, 90762 Fürth (DE)
(74) Representative: Banse & Steglich Patentanwälte PartmbB
(86) International application number: PCT/EP2017/070760
(87) International publication number: WO 2018/036879

(56) References cited:
- EP-A1- 1 902 739
- WO-A1-2009/073854
- US-A1- 2003 141 618

## Description

The invention relates to wound dressings comprising a polymer frame and at least one layer of preferentially parallel aligned fibers spanning the frame. The wound dressings may be used for eye treatment, especially for the treatment of the cornea. Furthermore, a method for manufacturing of the wound dressing by electrospinning is provided.

### Background of the invention

Wound dressings are materials that serve to protect wounds from negative environmental influence, for example bacterial infections or mechanical stress and promote wound healing. In general, wounds might have different causes such as accidental mechanical injury, chemical and thermal injury or injury in the course of surgery, for example the excision of tumors.

Modern wound dressings should allow good exchange of vapor and gases, possess good absorption capacity for water and toxins, for example endotoxins or inflammation mediators, serve as a framework for infiltration and growth of cells and positively influence cell growth and wound healing.

In the field of ophthalmology, amniotic membranes are most frequently used as wound dressings and implants for treating injuries of the ocular surface, especially to promote the healing process during corneal reconstitution after injuries of the cornea. The amniotic membrane is the innermost layer of the placenta and is derived from donors undergoing cesarean section, who are negative for communicable diseases such as HIV and hepatitis. Therefore, amniotic membranes have a limited availability and are relatively costly to prepare. Furthermore, diseases might be transmitted from the donor to the patient. The amniotic membranes are usually fixed to the eye by suturing the membrane to the ocular tissue. As amniotic membranes are obtained from natural sources, the quality of the product varies and is not easy to control.

As an alternative to membranes from natural sources, artificial membranes made from naturally occurring polymers have been developed. For example, a collagen-based, suturable membrane for ocular indications is available as Visu-Foil®. However, collagen is an expensive material and is relatively quickly degraded. Additionally, the use of artificial fiber-based biocompatible and/or biodegradable materials for use as wound dressing has been evaluated. For example, polycaprolactone (PCL) membranes obtained by electrospinning have been investigated as wound dressings.

WO 2015/092797 A1 discloses suturable multi-layer matrices comprised of biocompatible and/or biodegradable polymer fibers for use as wound dressings. Polymer fibers may, for example, be obtained by electrospinning.

Wound dressings comprising electrospun PCL membranes may additionally comprise active agents, for example the antibiotic tetracycline hydrochloride, which are released over a period of time, to promote wound healing (Chellamani et al., 2014).

Especially, parallel aligned fibers have raised interest for application in wound healing and tissue engineering, since they mimic the parallel aligned structure of some tissues, thus providing contact guidance and orientation to cells, to promote cell growth, tissue repair and healing.

Neither WO 2015/092797 A1, nor Chellamani et al. (2014) disclose parallel fiber structures that promote wound healing.

The generation of aligned polycaprolactone (PCL) fibers by electrospinning is, for example, disclosed by Doergens et al (2015). The document discloses the generation of PCL fibers by electrospinning and collecting the generated fibers on a rotating collector. The obtained fiber layers reveal a highly aligned orientation.

Salehi et al. (2014) discloses the generation of aligned poly(glycerol sebacate (PGS)/polycaprolactone (PCL) nanofiber scaffolds by electrospinning and investigates the physical properties of these membranes for diverse tissue engineering applications, especially for cornea treatment.

US 2014/0030315 A1 discloses the use of aligned nanofiber layers from chitosan as wound dressings and suggests a use on the corneal surface.

EP 1 902 739 A1 discloses the use of parallel aligned biopolymer fiber layers for treating damaged ocular tissue. The aligned fiber layers are organized in a multi-layer structure, wherein the orientation in at least one fiber layer differs from that in at least a superior or inferior layer to mimic the structure of corneal tissue. The fibers are prepared by *in vitro* assembly of highly oriented collagen in aqueous solution mediated by a strong magnetic field. The reaction is carried out in a "holder", which is a glass or plastic container comprising a polymerization solution and the fiber precursors. The holder is only a tool in the production method and not a component of tissue-like fiber product. Such processes based on *in vitro* polymerization are generally complicated and difficult to control, because polymerization must coincide with formation of the desired fiber macrostructure. Since polymerization and fiber formation depend from each other, minor variations can significantly affect the overall structure. Further, possible applications are very limited because of the specific properties of collagen.

However, Salehi et al. (2014), US 2014/0030315 A1 and EP 1 902 739 A1 do not disclose fixing the parallel aligned fiber structure to maintain its beneficial effect. Especially in ocular use, where the fiber layers are subjected to mechanical forces by the movement of the ocular bulb and the palpebral, the parallel structures can be easily destroyed. Furthermore, no means for permanently attaching the fiber layers to the eye is disclosed.

US 2015/0024493 A1 relates to the use of aligned fiber layers for small-diameter vascular prostheses. To fix the fibers in the parallel orientation, a binding agent is applied to the aligned fibers, to bond the fibers into the aligned arrangement and to obtain aligned fiber mats. Although the technology enables fixing the parallel structure, adding a binding agent is likely to reduce porosity of the fiber layers by filling the voids between the fibers.

WO 2009/073854 A1 discloses hemostatic bandages comprising nanofibers. The bandages comprise nanostructures for inducing blood coagulation. The nanostructures may be incorporated into the bandages by coating. In a proposed coating method, the fiber substrate is mounted in a frame, where after the fibers are coated and dried. The frame is only a tool in the coating method and not part of the hemostatic bandage.

US 2003/0141618 A1 discloses a method for producing a thin layer of oriented fibers, in which a solution of self-assembling monomer units, such as collagen units, is dispensed onto a rotating plate where it assembles into fibers. As noted above, such processes, which are based on *in vitro* polymerization, are generally complicated and difficult to control, because collagen assembly must be synchronized with formation of the desired macrostructure. Thus, minor variations can significantly affect the overall structure. Further, possible applications are limited because of the specific properties of collagen.

While wound dressings comprising parallel aligned fibers are known in the art, they generally have only insufficient mechanical stability of the fiber layers and there are problems in fixing the biologically advantageous parallel orientation of the fibers without substantially impairing the structure and porosity of the fiber layers. Furthermore, the wound dressings should be optimized for permanently attaching the dressings to the underlying tissue, for example to the cornea.

### Problem underlying the invention

Generally, it is a problem underlying the invention to provide dressings which overcome the above mentioned problems. Specifically, the problem is to provide a wound dressing for eye treatment, especially treatment of the cornea, having improved properties. Especially, the dressing should have good stability and the application to the patient should be convenient. More specifically, the problem underlying the invention is to provide a wound dressing, comprising at least one layer of nanofibers, which has an improved mechanical stability, wherein the improved mechanical stability enables adhering the wound dressing to a tissue by suturing or stapling. In a further aspect, the preferably parallel aligned orientation of the fiber layer shall be fixed without impairing the porous structure of the fiber layer. Furthermore, it is a problem underlying the invention to provide a simple and reproducible process for manufacturing the wound dressing.

### Disclosure of the invention

Surprisingly, it was found that the problem underlying the invention is overcome by the wound dressings and methods according to the claims. Further embodiments of the invention are outlined throughout the description.

The present invention relates to wound dressings, comprising a frame, which comprises at least one organic frame polymer and at least one layer of fibers spanning the frame, the fibers comprising at least one organic fiber polymer.

In the context of the present description, the term "fiber(s)" generally refers to polymer fiber(s).

A "fiber spanning the frame" contacts the frame in at least two sites of the frame and traverses the void surrounded by the frame between these two sites of contact. Thus, the fibers spanning the frame comprise a periphal region contacting the frame and a central region which is not contacting the frame.

Within the context of the present invention, the term "fiber layers" does not comprise textiles, especially no woven fabrics.

In a preferred embodiment, the wound dressing is for eye treatment, more preferably for treatment of the cornea. In the context of the present invention, the term "wound dressing" includes grafts, suitable for implantation into or onto the body, especially into or onto the eye. In a highly preferred embodiment, the wound dressing may be a graft used as artificial cornea replacement. Although the implant can be used for animals, especially mammals, in general, use for humans is highly preferred, especially for the human eye. A wound dressing is designed to be in direct contact with the wound. Thereby, it is distinguished from a bandage, which is generally used to hold a dressing in place. As used herein, a wound is a body surface, such as skin or cornea, which is not in its normal, healthy state. For example, it may be torn, cut, or punctured.

Preferably, the fibers in the at least one layer of polymer fibers spanning the frame are substantially parallel aligned. Within the context of the present invention, the term "substantially parallel aligned" refers to an organization of polymer fibers, wherein the longitudinal direction of less than about 40%, preferably less than about 30%, most preferably less than about 10% of the fibers in a layer deviate from the average longitudinal direction of the fibers in a layer by less than 23%, as determined by a top view on the layer of polymer fibers spanning the frame. The longitudinal direction of the fibers can be determined by analyzing the fiber layer by scanning electron microscopy and subsequent software based analysis of the obtained micrographs as for example disclosed by Doergens et al. (2015).

Preferably, the at least one layer of fibers spanning the frame is permanently adhered to the frame. The different embodiments of attaching the fiber layers to the frame are disclosed below in more detail.

As disclosed above, a parallel fiber organization promotes wound healing. By providing the layers of parallel fibers in a frame, this advantageous structure can be permanently preserved, while the porosity of the fiber layers is maintained over the whole area, where the fiber layer spans the void surrounded by the frame. Furthermore, the mechanical stability of the fiber layer is improved by the frame. Preserving the parallel structure and improving the mechanical stability is especially advantageous in applications, were the wound dressing is repeatedly subjected to mechanical forces, for example by the movement of the ocular bulb and the palpebral in ocular use of the wound dressing.

The wound dressing can be held in place during the application of the wound dressing, by holding the frame with forceps or other suitable devices. This is especially advantageous in an application wherein the dressing is applied by suturing or stapling. Consequently, the frame improves the handling of the wound dressing during application.

The term "about" could refer to a range of +/- 10%, preferably +/- 5% of the recited number or the recited number itself.

In a preferred embodiment, the layers comprise at least one layer of aligned fibers and at least one layer of non-aligned (random) fibers. Generally, it is preferred that the outer layer, which is directed away from the body surface, comprises random fibers. This is advantageous, because an outer layer of random fibers may stabilize aligned fibers in layers underneath. Preferably, the non-aligned fibers have an average diameter which is smaller than the diameter of the aligned fibers. Thereby, local voids in an underlying aligned fiber layer can be covered or filled with the finer random fibers, thereby stabilizing the overall structure. Preferably, the average diameter of the random fibers is at least 2 times smaller, preferably about 2 to 10 times smaller, than the average diameter of the aligned fibers.

Preferably, the fibers are obtained by electrospinning. Preferably, all the fibers in the wound dressing are obtained by electrospinning. Preferably, at least one layer of fibers spanning the frame, more preferably all the layers of fibers spanning the frame are produced by electrospinning.

As laid out in the background section above, aligned fiber layers can be obtained by electrospinning, which will be explained in more detail below. It is therefore a highly preferred embodiment of the present invention that all or at least some of the polymer fiber layers spanning the frame were obtained by electrospinning. In a less preferred embodiment, the polymer fiber layers, or at least one or more of the polymer fiber layers, may be obtained by other known methods, such as wet spinning, dry spinning, melt spinning, melt blow spinning or gel spinning.

In a further preferred embodiment of the present invention, the wound dressing comprises at least two stacked layers of fibers spanning the frame. The frame might be spanned by a stack of at least two layers of fibers on one side of the frame or on both sides of the frame. Within a stack of fiber layers, at least the fiber layer directly facing the tissue when the wound dressing is applied to a tissue, comprises substantially parallelly aligned fibers. The other fiber layers comprised in the stack of fiber layers may consist of, or comprise unaligned fibers. The wound dressing may comprise about 5 to about 1000, preferably about 5 to about 100, more preferably about 5 to about 30, and most preferably 7 to 8 stacked layers of fibers spanning the frame.

The fibers in at least one fiber layer may be substantially parallelly aligned in a different direction in relation to at least one other fiber layer. The term "Substantially parallel aligned in different direction" refers to an arrangement of the fiber layers, wherein the average longitudinal direction of the fibers in one layer deviates from the average longitudinal direction of the fibers in at least one other layer by at least more than about 10°, about 20°, about 30°, about 40°, about 50°, about 60°, about 70° and/or about 80°. By stacking or overlaying the fiber layers which are substantially parallelly aligned in different directions, an overall net-like structure is obtained. This net-like structure significantly improves the mechanical stability of the aligned fiber layers.

It is an advantageous property of the described stacked arrangement of layers that the superior biological properties of aligned fiber layers are maintained, while physical stability of the wound dressing is further improved.

As used herein, the term "layer" refers to a region, in which fibers have been deposited in a single spinning step. Different layers may be distinguishable by microscopical analysis of cross-section cuts. However, superimposed layers in the wound dressing may not be clearly distinguishable from each other. For example, when a layer of fine random fibers is deposited over a layer of larger fibers, at least a portion of the fine fibers may fill voids between the larger fibers, such that the layers penetrate each other at least in part.

Preferably, the inventive wound dressing is characterized by an overall flexibility, allowing for the adaptation of the shape of the wound dressing upon application of the wound dressing to differently formed surfaces. Especially the flexibility of the wound dressing allows for an adaptation to the curved shape of the eye, especially the cornea.

In a preferred embodiment, at least the central region of the wound dressing, which does not contact the frame, and/or the at least one fiber layer spanning the frame, is substantially transparent, which facilitates the use of the wound dressing for cornea treatment. More preferably, the overall wound dressing is substantially transparent. Preferably, the transparency of visible light (in the range of 400 nm to 800 nm) is at least 50%, preferably at least 60% or at least 70%. Preferably, this means that the transparency is not lower in any wavelength of this range.

In a preferred embodiment, the frame polymer and/or the fiber polymer is a biodegradable polymer and/or a biocompatible polymer. Within the context of the present disclosure, the term "polymer" encompasses different types of polymers such as for example homopolymers and co-polymers.

The term "biodegradable" refers to the property of a material to be able to be broken down or decomposed by microorganisms or within the body of an animal, especially when used as a wound dressing in a human, by physiological processes within a relative short time (within 1 to 18 months). Biodegradable medical devices implanted into the human body are usually not excised from the human body, but are maintained in the body until decomposition of the device under physiological conditions.

The term "biocompatible" refers to the ability of a material to perform its desired function with respect to a medical therapy, without eliciting any undesirable local or systemic effects in the recipient of the material, but generating the most appropriate beneficial cellular or tissue response. Especially, a biocompatible material is not rejected by the human immune system.

The organic frame polymer and/or the organic fiber polymer may be selected from the group consisting of, but not limited to, polyesters, polyethers, polyaminoacids, polyacrylates, acrylamide polymers, acrylate polymers, silicones, polypeptides, polysaccharides and/or polyolefins.

The organic frame polymer and/or the organic fiber polymer may for example be selected from the group consisting of, but not limited to, polymers and oligomers of glycolide, lactide, polylactic acid, polyesters of alpha-hydroxy acids, including lactic acid and glycolic acid, such as the poly(alpha-hydroxy) acids including polyglycolic acid, poly-DL-lactic acid, poly-DL-lactic acid, and terpolymers of DL-lactide and glycolide; ε-caprolactone and ε-caprolactone copolymerized with polyesters; polylactones and polycaprolactones including ε-polycaprolactone (PCL), poly(δ-valerolactone) and poly(gamma-butyrolactone); polyanhydrides; polyorthoesters; other hydroxyl acids; polydioxanone; polysaccharides, such as chitosan, and other biologically degradable polymers that are non-toxic or are present as metabolites in the body. Examples of polyaminoacids include, but are not limited to, polylysine (PLL), poly L-aspartic acid, poly L-glutamic acid, and styrene-maleic acid anhydride copolymer. Examples of derivatives of polyethylene glycol includes, but are not limited to, poly(ethylene glycol)-di-(ethylphosphatidyl) (ethylene glycol)) (PEDGA), poly(ethylene glycol)-co-anhydride, poly(ethylene glycol)co-lactide, poly(ethyleneglycol)-co-glycolide and poly(ethylene glycol)-co-orthoester. Examples of acrylamide polymers include, but are not limited to, polyisopropylacrylamide, and polyacrylamide. Examples of acrylate polymers include, but are not limited to, diacrylates such as polyethylene glycol diacrylate (PEGDA), oligoacrylates, methacrylates, dimethacrylates, oligomethoacrylates and PEG-oligoglycolylacrylates. Examples of carboxyalkyl cellulose include, but are not limited to, carboxymethyl cellulose and partially oxidized cellulose.

In an alternative embodiment of the wound dressing according to the present invention, the frame polymer and/or the fiber polymer is a biostable polymer. As used herein, "biostable" refers to polymers which are not broken down or decompose by microorganisms or within the body of an animal over an extended period of time of several months or years. Examples of biostable polymers include, but are not limited to, silicones, polyurethanes, polyethylenes, polysulfones, polyisobutylene, poly-4-methylpentene, polypropylene, polyvinylethylene, polybutylene, polydodecyl methacrylate, polyethlyene terephthalate, ethylene vinyl acetate, ethylene vinyl alcohol copolymer, polyethylene oxide, combinations thereof or copolymers thereof.

Since the main aspect of the present invention relates to the use of fibers generated by electrospinning, in a preferred embodiment, the organic fiber polymer is selected from a polymer suitable for processing by electrospinning.

Preferably, the organic frame polymer and/or organic fiber polymer is selected from the group consisting of elastin, collagen, polyurethane, ε-polycaprolactone (PCL), polylactide, polyglycolic acid, mixtures of these, and copolymers of these. These polymers are suited for electrospinning.

In general, a mixture of the above-mentioned polymers may be used in accordance with the present invention.

Most preferably, the polymer is PCL. The PCL used in the wound dressing of the present invention may have an average molecular mass of about 20 kDa to about 180 kDa, preferably from about 40 kDa to about 90 kDa, more preferably from about 70 kDa to about 85 kDa and most preferably about 80 kDa.

Different polymers may be selected as organic frame polymer or organic fiber polymer. Also, the different fiber layers may comprise different organic fiber polymers. However, in a highly preferred embodiment of the present invention, the organic frame polymer and the organic fiber polymer are the same polymer. It is also preferred that the different fiber layers are comprised of the same organic fiber polymer. Preferably, the wound dressing consists of one polymer material. Preferably, this one polymer is a biodegradable polymer, most preferably PCL.

It is a highly advantageous aspect of the wound dressing consisting of one biodegradable polymer, that the entire wound dressing can be degraded by the human body. Thus, no excision or detachment of the wound dressing or parts of the wound dressing is required after the wound has healed.

The fibers employed in the wound dressing of the present invention may be microfibers or nanofibers. Microfibers are fibers with an average diameter in the micrometer range. Accordingly, nanofibers are fibers with an average diameter in the nanometer range. Preferably, the fibers have an average diameter between about 100 nm and about 1,500 nm, preferably between about 200 nm and about 1,200 nm, more preferably between about 400 nm and about 1,000 nm, most preferably between about 500 nm and 900 nm.

According to the present invention, the at least one layer of fibers is porous. In the embodiment wherein several layers of fibers are stacked, it is a preferred embodiment that the stacked fiber layers also constitute an overall porous stack of layers. "Porous" layer or layers comprise voids (spaces where no polymeric material or other material is present) connecting the sides of the layers, thereby enabling an exchange of fluid or gas between the sides of the layer or layers. Thus, the overall wound dressing is porous in the axial direction, thereby allowing exchange of air and liquid between the wound and the environment.

In addition to the polymer material, the fiber layers may comprise at least one additional compound, especially at least one active agent. The at least one active agent may especially be selected from small molecular weight compounds, polypeptides and proteins.

The active agent may, for example, be a pharmaceutically active agent and/or an active agent that promote cell growth, adhesion or differentiation. In general, such active agent may improve wound healing. Preferably, the pharmaceutically active agent may be selected from agents which are typically used for ophthalmological applications, for example from anti-microbial agents, anti-bacterial agents; especially gentamicin or penicillin; anti-inflammatory agents, anti-viral agents, anti-fungal agents, growth factors; for example epidermal growth factor or platelet lysate; anti scarring agents; for example rosiglitazone; substances against dry eye disease; for example hyaluronic acid, tacrolimus, diquafosol; and/or substances hindering the degradation of the extracellular matrix; for example cacicol. The active agents are released from the wound dressing under physiological conditions after the wound dressing has been applied to the wound.

In a preferred embodiment, the wound dressing comprises a hydrogel. Preferably, the hydrogel comprises at least one active agent, preferably an active agent selected as outlined above. The hydrogel may be comprised in form of at least one additional layer on top or between the layers of fibers. Alternatively, the hydrogel may be used to fill the voids of the porous structure of the wound dressing. Preferably, the complete wound dressing is embedded in the hydrogel. In other words, the hydrogel may form a matrix in which the porous fiber structures are enclosed. The term "hydrogel" refers to a cross-linked network of hydrophilic polymers which are capable of absorbing high amounts of water. In particular, the cross-linked polymer hydrogels are capable of absorbing an amount of water in excess of 10 times their dry weight. The hydrogel may be a biodegradable and/or a non-biodegradable hydrogel. The polymer comprised in the hydrogel may be selected from chitosan, alginates, poly(ethylene glycole), and/or poly(ethylene oxide), which are examples for typical hydrogels used for medical applications. The hydrogel may comprise particles, including particles of at least one hydrogel different from the surrounding hydrogel or particles comprising at least one active agent. By varying the degree of polymer cross-linking, the release kinetics of active agents from the wound dressing may be varied. A higher degree of cross-linking will result in a slower release of the active agents, whereas a lower degree of cross-linking will result in a faster release of the active agents. Before application of the hydrogel to the wound dressing, it may be hydrophilized to enable or improve water absorption. For example, a polyester can be pre-treated with a base in order to cleave a portion of the ester bonds.

In general, a frame is an entity that surrounds a central void. In the inventive wound dressing, the frame surrounds a void or central region, which is spanned by the fiber layers. The frame is circumferential. Preferably, its lateral outer end defines the end of the wound dressing in all lateral directions. The frame shall confer stability to the fibers and fiber layers spanning the frame. Preferably, the frame is substantially flat, i.e. arranged in a plane. The frame may have any desired shape, which defines the shape of the wound dressing. Depending on the desired application, it may be round, oval, rectangular, or may have an irregular shape. For corneal wound dressings, the frame is preferably round.

The frame is an integral component of the wound dressing. Preferably, the wound dressing and the frame are elastic and not rigid. Thus, the wound dressing, including the frame, preferably has a soft structure. It can be bent and can adapt the form and shape of the body surface to which it is applied. The frame is not removed before applying the wound dressing to the wound. It is not a tool, which would only be mounted temporarily to the wound dressing in the production process.

The frame of the wound dressing may consist of a polymer film. Alternatively, the frame may consist of or may comprise randomly oriented fibers, such as a non-woven fiber material. Non-woven fiber materials with various fiber-diameters may be obtained by known methods, including wet fiber spinning, dry fiber spinning, melt fibers spinning, melt blow fiber spinning, gel spinning and electrospinning. In some cases, the fibers may be bonded after spinning. The bonding of the fibers may be performed by thermobonding, for example with head sealers or calenders, hydroentanglement, ultrasonic pattern bonding, needlepunching, chemical bonding, and other methods known in the art.

In a highly preferred embodiment, the frame is produced by electrospinning, preferably by electrospinning random fibers. After spinning a fiber layer, the frame can be excised from the layer in a desired form, for example by cutting or punching. Preferably, the step of producing the frame by electrospinning is integrated in the overall electrospinning process, which includes spinning the fiber layers spanning the frame. This is advantageous, because a frame can be prepared which is highly compatible with the fiber layers. Preferably, all fibers of the wound dressing are produced in a single electrospinning process.

The size and the form of the frame may be selected depending on the desired application area of the wound dressing. Preferably, the frame has the size and form suitable for application of the wound dressing to the human eye, especially to the cornea. Most preferably, the frame has the form of a ring.

In a preferred embodiment of the invention, the shape of the wound dressing is convex and may preferably be configured to cover an average cornea, preferably a human cornea. The eye as an organ has a complex geometry with the cornea resting as a dome on top of the eyeball. The dome of the human cornea covers an elliptical area with a mean horizontal meridian of 11.7 mm and a mean vertical meridian of 11.6 mm. The radius of curvature varies between 7 mm and 8.5 mm. This macroscopic shape of the cornea is taken into account in the wound dressing configured to cover an average cornea. Especially, the inventive wound dressing may cover an elliptical area with a mean horizontal meridian of about 11.7 mm, a mean vertical meridian of about 11.6 mm, and a radius of curvature of between about 7 mm and about 8.5 mm. Advantageously, a convexly shaped wound dressing reduces the risk of folds during the application of the wound dressing to the cornea. Due the curvature fitting of the wound dressing, it is potentially possible to attach the wound dressing sutureless due to the high adhesion.

A wound dressing of the described shape may be obtained by depositing the frame and/or the polymer fibers on molds shaped like an average cornea. Furthermore, a mold based on the specific shape of the cornea of a patient may be manufactured in order to create a wound dressing with a perfect fit to the patient's eye.

In a preferred embodiment of the invention, the frame is suitable for suturing or stapling the frame to a biological tissue. Thus, the thickness and width of the frame may be selected by the person skilled in the art to satisfy this requirement. Within the context of the present application, the term "suitable for suturing" refers to the property that a needle with a thread can pass through the frame without causing a rupture or tearing of the frame when the needle passes the frame or during the time when the thread adheres the frame to an underlying tissue. Likewise, "suitable for stapling" refers to the property that a stapler can pass through the frame without causing rupture or tear of the frame.

The advantageous suitability of the described wound dressing for suturing or stapling is facilitated by the frame of the wound dressing, which enables fixation of the wound dressing to an underlying tissue without disturbing or even destroying the structural integrity of the parallel fiber layer.

In a specific embodiment, the inner diameter of the ring is between about 3 mm and about 20 mm, and the outer diameter of the ring is between about 5 mm and about 25 mm, preferably the inner diameter is about 10 mm and the outer diameter is about 14 mm. Preferably, the resulting width of the ring is between about 0.5 mm and about 5 mm, more preferably between about 1 mm and about 2 mm.

In a preferred embodiment of the present invention, the fibers are permanently adhered to the frame. "Permanently adhered" fibers are connected to the frame under the usual conditions of use and over the average lifetime of the wound dressing. Preferably, the fibers are adhered to the frame by means of an adhesive, by welding or most preferably by embedding the fibers in the material constituting the frame. The fibers might be adhered to the frame by applying an adhesive composition. A suitable adhesive composition can be selected by the person skilled in the art depending on the nature of the fiber polymer and the frame polymer. Welding of fibers to the frame may be achieved by heat treatment, which leads to a partial melting of the fibers and/or the frame, thereby permanently adhering frame and fibers.

In a preferred embodiment, the layer or layers comprising aligned fibers are embedded in the material constituting the frame to adhere the fibers to the frame permanently. In this specific embodiment, the organic fiber polymer and the organic frame polymer are the same polymers. The embedding of the fibers in the material constituting the frame may be achieved by subjecting the contact sites of the fibers with the frame with a suitable organic solvent comprising the respective polymer and, partially solubilizing the polymer of the fibers and the frame and partially mixing the fiber polymer and the frame polymer at the contact site. After evaporating the solvent, the fiber material is thus embedded in the material constituting the frame.

In a further aspect, the present invention relates to a kit comprising the wound dressing described above and instructions for using the dressing. Furthermore, the kit might comprise a surgical needle and/or thread to be used for applying the wound dressing.

Subject of the inventions is also a process for manufacturing a wound dressing of the invention, comprising the steps of
(b) providing the frame (1) which comprises the organic frame polymer, and
(c) depositing the at least one layer of polymer fibers (4) on the frame (1).

In step (b), providing the frame may comprise electrospinning the frame. Then, the frame consists of or comprises electrospun fibers. In this embodiment, preferably a frame layer is produced by electrospinning, followed by converting the frame layer into the frame, for example by cutting or excising the frame.

In step (c), the layer is preferably deposited on the frame by electrospinning. Thus, the frame is provided as a substrate, on which the fibers for the layer are electrospun. Alternatively, the fiber layers can be formed separately and combined with the frame after electrospinning.

After step (c), the process may further comprise a step of:
(d) permanently adhering the fibers (4) to the frame.

In another embodiment, in step (c) the layer of polymer fibers (4) is deposited on the frame (1) by moving the frame (1) through the layer of fibers (4) generated by electrospinning. This embodiment is especially preferred if the frame is not obtained by electrospinning, but by different means, for example from a polymer film.

The present invention furthermore relates to a process for manufacturing a wound dressing as described above, comprising the steps of
(a) generating at least one layer of polymer fibers comprising an organic fiber polymer by electrospinning,
(b) providing the frame which comprises an organic frame polymer, and
(c) depositing at least one layer of aligned polymer fibers on the frame.

In this embodiment, step (a) may be carried out before or after step (b). Preferably, several layers of aligned polymers are deposited on the frame in step (c).

The generation of fibers by electrospinning is a process known in the art (Doergens et al. (2015), WO 01/27365 A1, US 2014/0046236, WO 2015/092797 A1). As used herein, the term "electrospinning" refers to a technology which produces fibers from a polymer solution or melt. Preferably, the fibers are generated from a polymer solution. In the process, the polymer solution or melt, is placed in a dispenser, and the generated fibers are collected by a collector. The dispenser is connected to a source of high-voltage, preferably of positive polarity, while the collector is grounded. Thereby an electrostatic filed is induced between the dispenser and the collector. At a critical voltage, the charge repulsion begins to overcome the surface tension of the solution or melt, generating a jet that travels from the dispenser to the collector. While the jet travels from the dispenser to the collector, the organic solvent evaporates from the polymer solution, generating a solid fiber. Likewise, a polymer melt cools and thereby solidifies when traveling from the dispenser to the collector. The dispenser may, for example, be a syringe with a metal needle.

Electrospinning confers a unique and advantageous structure to a fiber product, which is different from products obtained by other known spinning techniques. The fiber structure is a result of movements of a liquid polymer raw material guided by an electric field, during which the solvent is evaporated and the fibers are formed. Electrospun fiber layers are characterized by branching points between fibers (which result from splitting up of liquid drops in the electrical field) and slight variations of the fiber diameter. As desired in a specific layer, highly aligned fibers or a dense network of connected fibers is obtainable thereby.

Aligned fibers may be obtained by using a rotating collector, for example a rotating mandrel, a rotating disk or non-solid rotating devices such as a rotating circle with copper wires or metal frame collectors. Thus, it is a preferred embodiment of the process that during electrospinning, the fibers are collected on a rotating device. In a further preferred embodiment, a layer of aligned fibers is generated by collecting the electrospun fibers on a rotating device. Preferably, an electrospinning apparatus which includes a rotating device as disclosed in Doergens et al. (2015) is used. The electrospinning apparatus disclosed by Doergens et al. (2015) comprises a syringe pump with an adjustable pump rate, a syringe with an outlet consisting of a conductive material, a high voltage source and a collector which consists at least partially of a conductive material. The syringe or capillary and the collector are connected via the high voltage source.

The employed solvent may be a mixture of different organic solvents. Preferably, a mixture of chloroform and ethanol (9:1 v/v) is used. Alternatively, a mixture of di-chloromethane (DCM) and methanol (7:3 v/v) may be used.

Preferably, a solution of about 10% to about 20% by weight, more preferably about 12% to about 16% by weight, most preferably about 14% by weight of PCL is used.

The properties of the obtained fibers are influenced, amongst other factors, by the employed polymer, employed solvent, concentration of polymer solution, the applied voltage, the distance between dispenser and collector and the tangential rotation speed of the rotating collector.

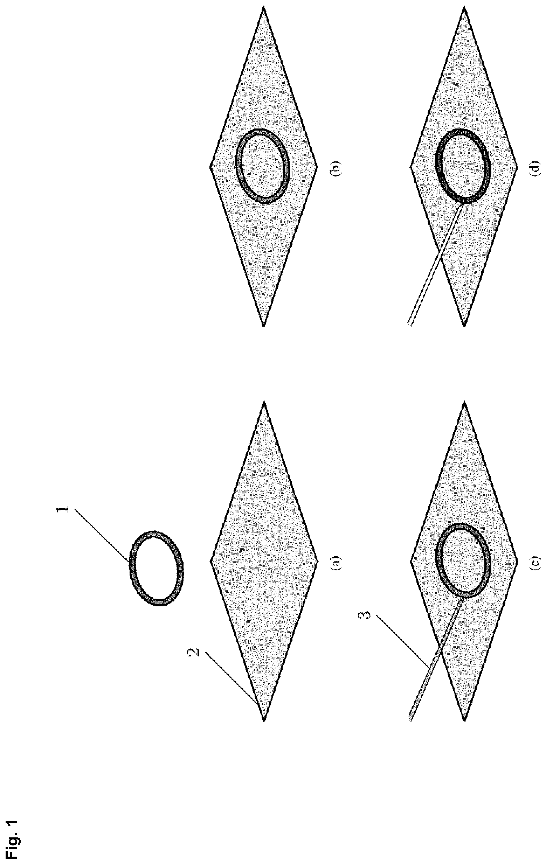

The generation of aligned polymer nanofibers by electrospinning is disclosed in detail by Doergens et al. (2015). Specific reference is made to sections 2.1. to 2.2. and Fig. 1, which disclose a process and a suitable apparatus for electrospinning PCL fibers.

The frame provided in step (b) of the inventive process may be obtained by casting a film of organic frame polymer, preferably PCL, and excising the frame, preferably in the form of a ring, from said casted film.

In a preferred embodiment of the inventive process, in step (c) the at least one layer of fibers is deposited on the frame by moving the frame through the layer of fibers generated by electrospinning. It is understood that moving the frame through the layer of fibers can be achieved either by actively moving a frame through a stationary fixed layer of fibers, or by actively moving a layer of fibers through a stationary fixed frame.

To enable a more convenient handling of the frame in step (c) of the process described above, prior to depositing layers of aligned fibers on the frame, the frames may be placed on a flat substrate. In an alternative preferred embodiment, the substrate may be in the form of a mold comprising at least one convex dome in form of a cornea as described above. Preferably, the mold comprises an array of several domes. Preferred substrates are polytetrafluorethylene (PTFE) sheets or glass. Subsequently, an evaporable liquid, preferably water, may be dispensed between the frame and the substrate to adhere the frame on the substrate by adhesive forces.

To obtain a frame, which is adapted for the manufacture of a convex wound dressing, a two-part mold may be used. The upper part of the mold comprises a concave dome, whereas the lower part of the mold comprises a corresponding convex dome. The frame polymer in form of a film or a non-woven fabric is moved between both parts of the mold and the mold is shut. Either by pressure, temperature or by a solvent atmosphere, the film or a non-woven fabric are shaped according to the geometry of the mold. The step of shaping the frame may be performed either before or after the frame is excised from the film or non-woven fabric. For example, the dome may have a radius of 8 mm while the covered area is a circle with a radius of 7 mm. The radius and area may be adjusted to the specific geometry of a patient's cornea in order to obtain a wound dressing which fits the cornea.

The deposition of several layers of fibers on the frame may be achieved by repeatedly moving the frame through the layer of fibers. To deposit different layers of fibers in different alignment orientations on the frame, the frame might be tangentially rotated between the different rounds of depositing fiber layers on the frame.

After step (c), the process may further comprise a step of cutting the fibers, which radially extend over the edge of the frame. Prior to cutting the fibers might be intermediately adhered to the frame by wetting the fibers.

In a further preferred embodiment, the inventive process furthermore comprises step d) of permanently adhering the fibers to the frame. The fibers might be permanently adhered to the frame by subjecting an adhesive composition to the contact site between fibers and frame or by welding the fibers to the frame. In a preferred embodiment, the fibers are permanently adhered to the frame by applying a solution of the fiber polymer solubilized in a solvent to the surface of the frame and subsequently evaporating the solvent. By subjecting the solution to the contact site of the frame and the fibers, the fibers and/or the frame may be partially solubilized. The choice of the organic solvent depends on the nature of the selected fiber polymer, for PCL chloroform is preferentially used. In a preferred embodiment of the process, wherein the fiber polymer is PCL, the polymer solution applied to the frame is a solution of PCL in an organic solvent. Preferably, the polymer solution has a viscosity of about 1 Pas to about 100 Pas, preferably about 10 Pas to about 50 Pas, most preferably about 20 Pas.

After permanently adhering the fibers to the frame, the frame may be detached from an underlying substrate by evaporating the liquid between the frame and the substrate.

A process for manufacturing a wound dressing according to the present invention is furthermore disclosed in Examples 1 and 2.

In the inventive process, it is advantageous that the fibers can be produced in a spinning process from a spinning solution comprising the fiber polymer, such as electrospinning. Preferably, the fibers are not produced in a process, in which a polymerization process occurs during spinning. EP 1 902 739 A1 discloses such a process, in which fibers are produced from solution by polymerizing collagen precursors. Such combined polymerization and spinning processes are generally more difficult to control, more error-prone and less efficient.

In a further aspect, the present invention relates to a wound dressing obtained or obtainable by the process as described above.

The inventive wound dressing may be sterilized prior to packaging and/or use.

In a further aspect, the present disclosure relates to the use of the wound dressing as described above for eye treatment and/or eye surgery, preferably of the cornea. Preferably, the use comprises suturing or stapling the wound dressing to a tissue, preferably a wound or to the cornea through the frame of the wound dressing.

When used in accordance with the present disclosure, the wound dressing may be withdrawn from the wound or cornea after a period of time. Preferably, the wound dressing is not withdrawn from the wound or the cornea and biodegraded on the wound or cornea after a period of time.

In a further aspect, the present disclosure relates to a method of treatment comprising the step of applying the wound dressing as described above to a tissue, preferably a wound tissue or a cornea of an animal, more preferably a human, most preferably a patient in need of a wound treatment. Preferably, the method of treatment comprises suturing or stapling the wound dressing to a tissue, preferably a wound tissue or to the cornea through the frame of the wound dressing.

Exemplified embodiments of the invention and aspects of the invention are shown in the figures.

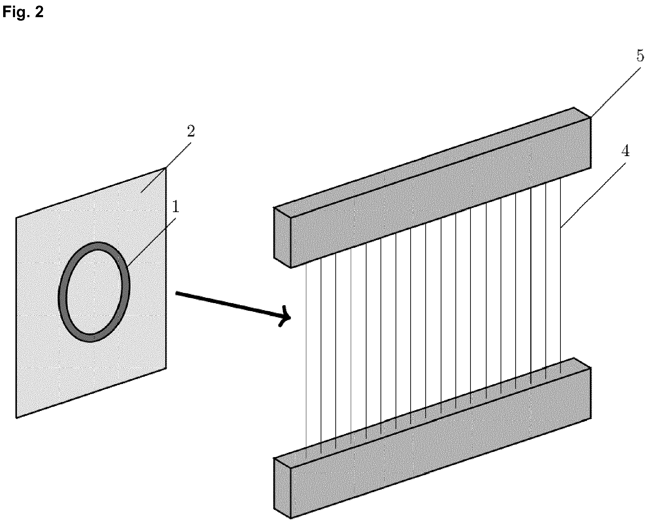

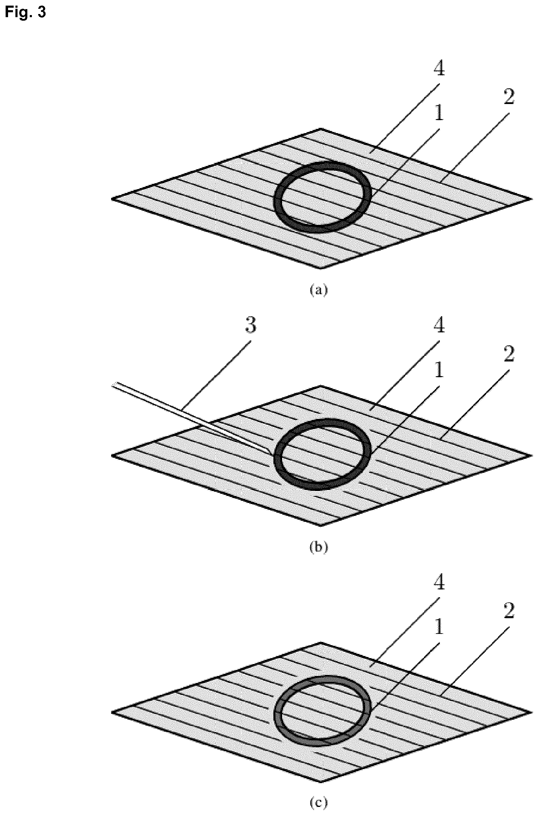

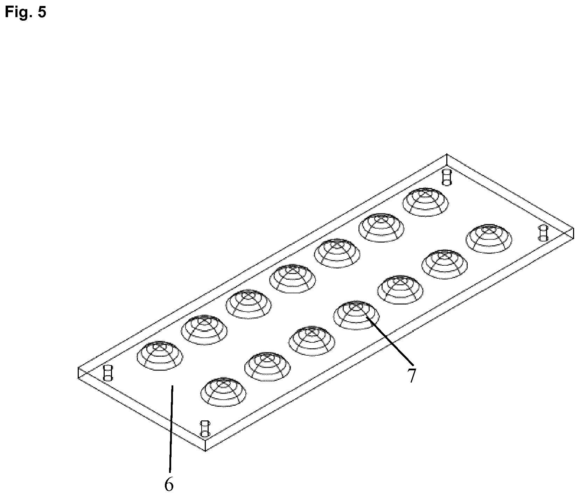

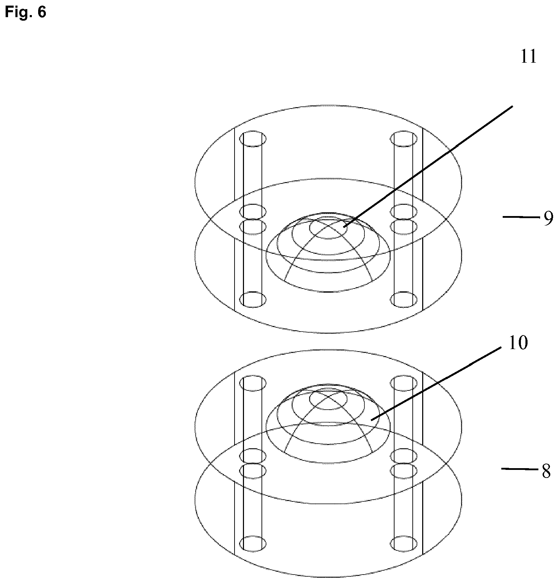

Fig. 1 schematically depicts the deposition of a polymer frame in the form of a ring (1) on a flat substrate (2). By adding a liquid between the ring (1) and the substrate (2) by means of a capillary (3), the ring (1) is attached to the substrate (2) by adhesive forces. As shown in Fig. 2, the ring (1) attached to the substrate (2) is moved through a layer of substantially parallel fibers (4) to deposit the fiber (4) layer on the ring (1). As described above, the fibers (4) were previously generated by electrospinning and collected on a rotating collector (5) to obtain a layer of substantially parallel fibers (4). After deposition of the substantially parallel fibers (4) on the ring (1) attached to the substrate (2), fibers (4) extending over the edge of the ring (1) are carefully cut using a capillary (3), as shown in Fig. 3. As shown in Fig. 5, the substrate may also be a mold (6) comprising an array of domes (7). To obtain rings that are adapted for producing convex wound dressings, a combination of a mold comprising a lower part (8) with a convex dome (10) and an upper part (9) with a concave dome (11), as depicted in Fig. 6, may be used.

The wound dressing according to the invention and the process for manufacturing the wound dressing solves the problem underlying the invention. By providing fiber layers in a frame and stacking the fiber layers, the mechanical stability of the wound dressing is substantially improved, while the porosity of the fiber layers is maintained. Furthermore, the advantageous structure of substantially parallel fiber layers, which promotes wound healing, is preserved. Preserving the parallel structure and improving the mechanical stability is especially advantageous in applications, were the wound dressing repeatedly subjected to mechanical forces, for example by the movement of the ocular bulb and the palpebral in ocular use of the wound dressing.

In addition, the frame comprised in the inventive wound dressing advantageously supports the fixation of the wound dressing to an underlying tissue by suturing or stapling, without disturbing or even destroying the structural integrity of the parallel fiber layer. Furthermore, the wound dressing can be held in place during the application of the wound dressing, especially if the dressing is applied by suturing or stapling, by holding the frame with forceps or other suitable devices. Consequently, the frame improves the handling of the wound dressing during application.

It is a further advantageous property of the inventive wound dressing, that the entire wound dressing can be degraded by the human body, rendering the excision of the wound dressing or parts of the wound dressing after healing unnecessary.
Figure 1 shows schematically the attachment of a polymer frame in form of a ring to a substrate using the adhesive force of water. (a) PCL ring and substrate; (b) PCL ring on substrate; (c) Capillary with water on the PCL ring; (d) Empty capillary and attached PCL ring.
Figure 2 shows schematically the deposition of the aligned fibers on the frame.
Figure 3 shows schematically the cutting of fibers and release of the PCL rings. (a) Substrate and ring after collecting fibers; (b) Cutting the fibers with the capillary; (c) Dried PCL ring with cut fibers.
Figure 4 shows micrographs of the fibers deposited on the ring.
Figure 5 shows a mold with an array of domes for the deposition of polymer fiber layers.
Figure 6 shows a two-part mold for the production on frames adapted to the cornea.
Figure 7 shows a domes-shaped collector of stainless steel with a diameter of 14 mm and a curve radius of 8 mm, which was used as a substrate for preparing the wound dressing in an electro-spinning method according to example 2.
Figure 8 shows a SEM picture at a magnification of 250X of the wound dressing produced according to example 2. The lighter upper section corresponds to the frame and the darker lower section corresponds to the central region with aligned fibers spanning the frame.
Figure 9 shows a SEM picture at a magnification of 20kX of the wound dressing produced according to example 2. The picture shows the central region comprising aligned fibers, which span the frame, and random fibers deposited thereon in a final step.

### Examples

### Example 1: Preparation of ring shaped wound dressing with aligned PCL fibers

### 1.1 Film cast

0.6 g of polycaprolactone (PCL) (Mn of 80.000) were dissolved in 10 ml chloroform (ACS grade) and stirred with 350 U/min in a fume cupboard for 2 hours at room temperature. Petri dishes with a diameter of 9 cm were cleaned using isopropanol (technical grade) and dust free tissues. The stirred solution was cast in the petri dishes in the fume cupboard and the glass covers of the petri dishes were placed on the top. After 48 hours in the fume cupboard, the glass covers were removed and the PCL films were removed from the petri dishes.

### 1.2 Ring Preparation

The PCL films were placed on a cutting-board. Using a set of two circular knives with the diameters of 14 mm and 10 mm, rings were cut out of the PCL films.

### 1.3 Fiber Spinning

A solution of 9 ml chloroform, 1 ml ethanol (ethanol 99% denatured with 1% MEK technical grade) and 1.4 g PCL was prepared and stirred with 350 U/min in a fume cupboard for 2 hours at room temperature. The solution was filled in a 10 ml all-glass syringe with Luer-Lock metal nozzle. A stainless steel needle with a diameter of 0.8 mm and a flat tip was attached to the syringe using the Luer-Lock. The remaining air in the syringe was removed by carefully releasing it through the needle.

The used electrospinning device consisted of a syringe pump with an adjustable pump rate, a high voltage source and a collector. In order to generate a high output of oriented fibers, a spinning wheel collector was chosen. The spinning wheel collector consisted of 8 metal bars held by two disks. The distance between from each bar to the next bar was 6 cm. The disks and so the bars could be rotated with up to 1000 U/min. The electrospinning device used in the experiment is disclosed in Fig. 1 of Doergens et al. (2015).

The syringe was placed and fastened in the syringe pump of the electrospinning device. Using a measuring tape, the pump was positioned inside the electrospinning device so the tip of the needle was in a distance of 15 cm to the outer edge of the bars of the collector. By attaching the alligator clamp attached connected to the end of the cable coming from the high voltage source to the Luer-Lock, the needle was connected with the high voltage source. A dispensing rate of 4 ml/h was set on the syringe pump and a voltage of 20 kV on the high voltage source. The collector was set on a rotating rate of 900 U/min. After one hour of electrospinning the collector was stopped, the voltage was removed and the syringe pump was turned off.

### 1.4 Fiber Harvesting

The PCL rings were placed next to each other on a flat substrate (A PTFE sheet with the thickness of 2.0 mm or a glass substrate for microscopy were used). Using a syringe, a little amount of water (on the 10 µl scale) was dispensed between the substrate and the ring in order to make the ring adhere on the substrate by using reversible adhesive forces. The process of attaching the rings to the substrate is shown in Fig. 1.

Some fibers at the side of the collector were destroyed in order to generate an access point for the substrate. The substrate was picked up with the tweezers and moved behind the fibers. By moving the substrate through the fibers, as shown in Fig. 2, the fibers were harvested and deposited on the rings. The process of collecting the fibers can be done repeatedly on several locations on the collector in order to generate thicker layers of fiber. If desired, the substrate can be rotated between different collection steps in order to generate layers comprising substantially parallel fibers which are oriented in different directions in the different layers.

After depositing the parallelly oriented fibers on the PCL rings and the substrate, the substrate was put down with the rings facing upwards. Using a syringe, the fibers close to the outer side of the ring were wetted. The fibers extending over the edge of the rings were carefully cut using either the sharp tip of the capillary or a scalpel. The rings were left on the substrate until the water had evaporated (about 30 minutes), subsequently the rings were removed from the substrate using tweezers. The progress of freeing the PCL rings is schematic shown in Fig. 3.

### 1.5 Optional: Connecting the rings and the fibers

In order to generate a more stable connection between the ring and the fibers, a PCL and chloroform solution with a viscosity of about 20 Pas (range of 1.0 Pas to 100.0 Pas) was prepared. A capillary was drawn from a Pasteur pipette using the flame of a Bunsen burner. The solution was carefully applied on top of the surface of the PCL ring covered with fibers. The fibers resting on the surface of the PCL ring and the surface of the PCL ring were partial dissolved. After the evaporation of the solvent, the ends of the fibers were embedded in the PCL ring.

### 2 Results

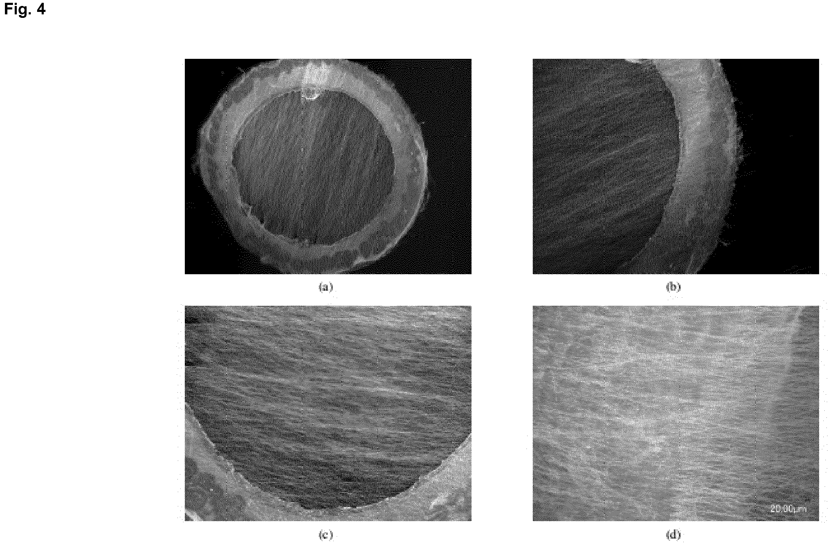

By the described procedure, a wound dressing as shown in Fig. 4 with different magnifications was obtained. The wound dressing contains several layers of aligned fibers deposited in different orientations. The beads on the fibers are a result of the electrospinning process. The generation of said beads can be avoided by optimizing the conditions of the electrospinning process.

The wound dressing obtained by the process as described in Example 1 exhibits a high mechanical stability and a high porosity in the axial direction of the wound dressing, which allows exchange of gas and fluids. Furthermore, the advantageous structure of substantially parallel aligned fibers, which promotes wound healing, is fixed by the permanent attachment of the fiber layers to the frame. The polymer frame of the wound dressing advantageously supports the fixation of the wound dressing to an underlying tissue by suturing or stapling, without disturbing or even destroying the structural integrity of the parallel fiber layer.

### Example 2: Electrospun scaffolds with a dome shape matching the cornea

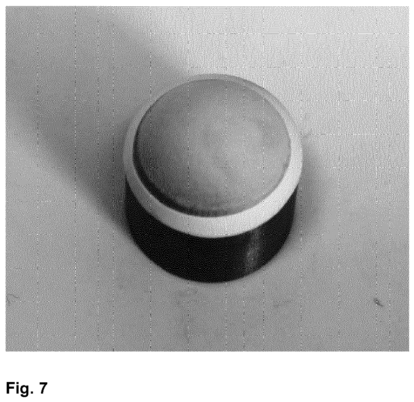

In order to produce scaffolds approximately matching the shape of a human cornea, domes of stainless steel with a diameter of 14 mm and a curve radius of 8 mm were manufactured (Fig. 7). The dome-collectors were conductive at the bottom for spinning random nanofibers directly on the dome shape. The aim was to generate a three layer nanofiber scaffold. The first layer was the frame, i. e. a ring of random nanofibers to provide the mechanical stability for suturing. The second layer was made from aligned nanofibers to provide an aligned structure and a higher transparency in the center of the scaffold. The third layer was made from random nanofibers to protect the aligned fibers from damage caused by being touched with tweezers or later on by the shear of the eyelid.

For the spinning of random fibers, 1.1 g PCL was dissolved in a mixture of 7 ml formic acid and 3 ml acetic acid. The mixture was stirred at 400 U/min for 4 hours at room temperature and normal atmosphere. Further, a solution of 1.4 g PCL solved in a mixture of 7 ml chloroform and 3 ml ethanol was prepared previously for spinning aligned fibers. The mixture was stirred at 400 U/min for 12 hours at room temperature and normal atmosphere.

The solutions were filled into a 10 ml syringe with a 0.8 mm stainless steel needle. The syringe containing the acid based solution was put in a syringe pump and placed in a distance of 12 cm from the needle tip to the dome-collector. The collector was placed in a holder (including a metal ring to increase the spinning surface to gain a more homogenous mesh) so the dome side was facing the needle, further the collector grounded at its bottom. A direct current at 17 kV was applied for 10 minutes while the pump was running at 0.2 ml/h. After 10 minutes of random nanofiber spinning the current was shut down. The dome was taken from the holder; the inner part of the spun nonwoven was cut with a 10 mm trepan, so a ring of a width of 2 mm remained on the dome.

The dome was placed in the holder again. A bar collector (distance between the bars 3 cm) was set up so the dome was located between the two bars. The bar collector was grounded while the dome stayed unconnected. The syringe was replaced with the one containing the chloroform based solution. The distance between collector and needle was enlarged to 20 cm and the pump rate raised to 4 ml/h. 20 kV have been applied on the needle for 10 minutes, then the power was shut down and the bar collector was carefully removed.

Again the syringe was replaced with the one containing the acid based solution. The bottom of the dome was grounded and the distance between collector and needle was reduced to 12 cm. A direct current of 17 kV was applied for 2 minutes while the pump was set on 0.2 ml/h. After the current was shut down, the dome was taken from the holder (Fig. 7) and the fibers on the sides were carefully removed using a scalpel. The fibers resting on top of the dome were wet with isopropanol and then carefully removed using tweezers. Even if the scaffold shown in Fig. 7 appears to be non-transparent, the nanofibers become transparent when being wetted as shown in Salehi et al., 2017.

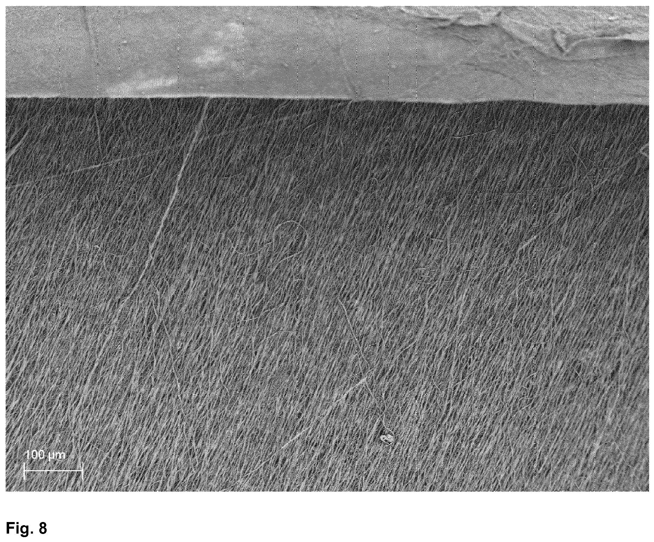

For analysis, the dome was carefully mounted on a SEM sample holder. The sample was sputter coated with gold before the SEM pictures were taken. Fig. 8 shows a SEM picture with a magnification of 250X. The lighter upper part of Fig. 8 is the ring of random nanofibers. It looks like a solid block, because the random nanofibers mostly have a diameter in the range 100 nm to 200 nm and so cannot be identified as single fibers at such a relatively low magnification. In contrast, the aligned nanofibers are clearly visible in the lower part of the picture. They form a lamellar structure. The aligned fibers have a diameter of approximately 500 nm and so are clearly visible at a magnification of 250X. The alignment of the fibers on Fig. 8 is still visible even if it is partially disturbed by putting the dome shaped sample on a flat disk shaped SEM sample holder.

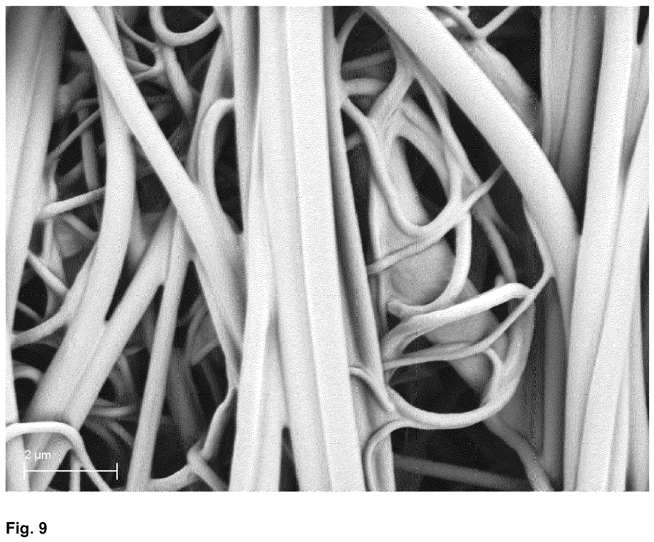

Using higher magnifications like 20kX, the random nanofibers are clearly visible (Fig. 9). As Fig. 9 shows, the random nanofibers are merging with some of the thicker random nanofibers at the contact points. This kind of merge is securing the aligned nanofiber layer on the random nanofiber layer.

### Example 3: Preparation of nanofiber hydrogel compounds

In a first step, nanofiber scaffolds of randomly fibers were prepared as described in example 1 or example 2. Fiber layers of random fibers of different thicknesses were prepared. Following to the preparation of the nanofiber scaffolds, these materials were used for the incorporation of hydrogels. In general, hydrophilic fiber materials can be used directly for incorporation of hydrogels. Hydrophobic materials, such as the PCL fiber scaffolds, have to be modified prior to the incorporation of hydrogels to increase the wetability. The modification of the PCL hydrophobic materials was done by different procedures. One method was the treatment of PCL with sodium hydroxide solution (1 M, for 1 hour) to break the ester bonds on the fibers surface. After hydrolysis, the nanofiber surface contains alcoholic and carboxylic groups, which enables an improved wetting and the incorporation of the hydrogel into the pores of the scaffolds. The samples were washed several times with deionized water before they were placed in a desired device for compounding, like a well plate or any other mold. The hydrogel was poured onto the modified or the unmodified hydrophilic samples, such that the scaffolds were infiltrated by the hydrogel. In case of alginate as hydrogel (i.e. 4 wt.%. medium viscosity sodium alginate in deionized water) the compound was dried after infiltration for 12 hours before crosslinking with CaCl₂ solution (i.e. 0,2 M CaCl₂ in deionized water) for 15 minutes. The final compound was obtained after cutting the sample into desired size. Hydrogels were prepared having average thicknesses of about 80 to 100 µm, but with varying fiber contents (see table 1; the fiber filled region of the compound in Vol-% corresponds to the thickness of the fiber layer in the hydrogel in µm).

### Example 4: Results

### 4.1 Suture-ability tests

The resistance of nanofiber hydrogel compounds having various nanofiber layer thicknesses, which were prepared according to example 3, was determined in the suture retention test described by Kueng et. al. (2016). As noted above, the hydrogels had average thicknesses of about 80 µm to 100 µm. They contained fiber scaffolds of various thicknesses. Therefore, the fiber content in the hydrogels varied from about 5 µm to 10 µm to about 30 µm to 45 µm (see table 1). The test was also carried out with an amniotic membrane and with a comparable scaffold without hydrogel.

Briefly, circular samples with a diameter of 14 mm were cut out of the specimens that should be analyzed. A suture was passed through the samples in a distance of 1 mm from the rim of the sample and knotted to a loop. The sample was clamped in a tensile testing machine, while the loop was fixed with a bolt in the second clamp of the tensile testing machine. The suture was pulled out of the sample with a deformation speed of 10 mm/min while the resisting force was measured. The maximum force was divided by the thickness of the samples at the puncturing point and given as suture retention strength. The results are summarized in table 1.

**Table 1: Results of suture-ability test with various PCL-fiber mesh scaffolds:**

| **Probe** | **Nanofiber filled region of the compound** | **Force / thickness ratio** |
|---|---|---|
| | **[Vol.- %]** | **[N/mm]** |
| amniotic membrane | - | ≈ 2,7 - 5,3 |
| fiber layer (dry) | - | ≈ 16,3 - 20,1 |
| fiber layer + hydrogel | 5-10 | ≈ 1,8 - 3,1 |
| fiber layer + hydrogel | 10-20 | ≈ 2,6 - 4,6 |
| fiber layer + hydrogel | 20-30 | ≈ 3,1 - 6,1 |
| fiber layer + hydrogel | 30-45 | ≈ 5,0 - 10,5 |

The results show, that the electrospun fiber layers with or without hydrogels have a high mechanical strength. Thus, they have an appropriate stability for wound dressing applications. Even a hydrogel comprising only about 10 to 20% fibers has a strength which is comparable to natural membranes.

### 4.2 Transparency

Wound dressings for eye treatment and corneal surgery should be sufficiently transparent. This is important for preserving clear vision of the patient. Further, it is important that the eye can be monitored from the outside, for example to determine if red stains or other discolorations are formed. For these reasons, the wound dressing should have a transparency as high as possible and at least of about 50%.

Transparencies of the PCL nanofiber hydrogel compounds (NFHC) were measured with a UV-Vis spectrophotometer through the wave length area of visible light (400 - 800 nm) with a measuring point each 5 nm according to Salehi et al., 2017. Nanofiber hydrogels were prepared in example 3 above, as used in the suture-ability test above. The compounds were cut into circular discs with a diameter of 14 mm and a total compound thickness of around 80 to 110 µm. These samples were placed in a 24-well plate and the transparency was measured with a micro plate reader. The results are shown in table 2 as mean of 3 samples for each fiber content.

**Table 2: Transparency of nanofiber scaffolds at 400 - 800 nm with different thicknesses in compound vs. transparency:**

| **Probe** | **Nanofiber filled region of the compound [Vol.- %]** | **Transparency [%]** |
|---|---|---|
| fiber layer + hydrogel | 5-10 | ≈ 68 - 93 |
| fiber layer + hydrogel | 10-20 | ≈ 59 - 86 |
| fiber layer + hydrogel | 20-30 | ≈ 57 - 86 |
| fiber layer + hydrogel | 30-45 | ≈ 54 - 86 |

The results show, that the compounds have sufficient transparencies for application as cornea wound dressings. The transparencies allow monitoring of the eye by the clinician and preserve vision of the patient.

### Example 5: Application of nanofiber hydrogel compound to a human cornea

To approve the applicability of the device in clinical surgery samples of the nanofiber, hydrogel compounds were prepared as described in example 3 having an average thickness of 80 to 100 µm, and comprising a random fiber layer of about 10 to 20 µm thickness. For testing the applicability a human cornea declared for scientific purposes was clamped in an artificial front chamber and inflated with PBS buffer. The cornea was placed under a light microscope before the compound was placed on top of the cornea. The surgery was performed by a medical doctor in ophthalmology with profound experience in corneal surgeries. The scaffold was sutured to the cornea with 8 single knots without disruptions.

The surgery confirmed the applicability in case of suture ability while being easy to handle for the surgeon. The hydrogel compounds could be fixed tightly on the cornea. Applicability for therapeutic applications was confirmed.

### Literature:

Chellamani KP, Vignesh Balaji RS, Veerasubramania D: Development of Wound Dressing Made of Electro Spun Tetracycline Hydrochloride Drug Incorporated PCL (Poly(ε-Caprolactone)), Nanomembrane International Journal of Emerging Technology and Advanced Engineering, (2014) 4, 251 - 256
Doergens A, Roether JA, Dippold D, Boccaccini AR, Schubert DW: Identifying key processing parameters for the electrospinning of aligned polymer nanofibres, Materials Letters, (2015) 140, 99-102
Küng F; Schubert DW, Stafiej P, Kruse FE, Fuchsluger TA, A novel suture retention test for scaffold strength characterization in ophthalmology, Material Science and Engineering C, Volume 69, 22 July 2016, Pages 941-946, ISSN 0928-4931, DOI: 10.1016/j.msec.2016.07.052
Salehi S, Fathi M, Javanmard SH, Bahners T, Gutmann JS., Ergün S, Steuhl KP and Fuchsluger TA,, Generation of PGS/PCL Blend Nanofibrous Scaffolds Mimicking Corneal Stroma Structure. Macromol, Mater. Eng. (2014) 299: 455 - 469
Salehi S, Czugala M, Stafiej P, Fathi M, Bahners T, Gutmann JS, Singer BB, Fuchsluger TA, Poly (glycerol sebacate)-poly (ε-caprolactone) blend nanofibrous scaffold as intrinsic bio- and immunocompatible system for corneal repair, Acta Biomaterialia, Volume 50, 1 March 2017, Pages 370-380, ISSN 1742-7061, DOI: 10.1016/j.actbio.2017.01.013.

## Claims

1. A wound dressing, comprising a frame (1), which comprises at least one organic frame polymer and at least one layer of fibers (4) spanning the frame (1), the fibers (4) comprising at least one organic fiber polymer.

2. The wound dressing according to claim 1, wherein the fibers (4) in the at least one layer are substantially parallel aligned.

3. The wound dressing according to any of the preceding claims, wherein the fibers were obtained by electrospinning.

4. The wound dressing according to any of the preceding claim, which comprises several layers of fibers (4) spanning the frame, wherein the fibers are substantially parallel aligned in different directions in different layers.

5. The wound dressing according to any of the preceding claims, wherein
the frame polymer and/or the fiber polymer is a biodegradable polymer; and/or
the frame polymer and/or the fiber polymer is selected from the group consisting of polyester, polyether, polyaminoacid, polyacrylate, acrylamide polymers, acrylate polymers, silicone, polypeptide, polysaccharide and/or polyolefin, wherein preferably the frame polymer and/or the fiber polymer is selected from elastin, collagen, polyurethane, ε-polycaprolactone (PCL), polylactide, and/or polyglycolic acid; and/or
the frame polymer and the fiber polymer are the same polymers.

6. The wound dressing according to any of the preceding claims, wherein the at least one layer of fibers (4) is porous.

7. The wound dressing according to any of the preceding claims, which comprises a hydrogel, preferably a hydrogel comprising at least one active agent.

8. The wound dressing according to any of the preceding claims, wherein the frame (1) consists of a polymer film or a non-woven fiber material, preferably in the form of a ring.

9. The wound dressing according to any of the preceding claims, wherein the shape of the wound dressing is convex, preferably configured to cover an average cornea.

10. The wound dressing according to any of the preceding claims, wherein the at least one layer of fibers spanning the frame is permanently adhered to the frame.

11. The wound dressing according to any of the preceding claims, wherein the layer or layers of fibers (4) spanning the frame (1) are embedded in the material constituting the frame (1).

12. The wound dressing according to any of the preceding claims, wherein the wound dressing is for eye treatment, preferably for treatment of the cornea, and/or eye surgery, preferably for surgery of the cornea.

13. A process for manufacturing a wound dressing of the preceding claims, comprising the steps of
(b) providing the frame (1) which comprises the organic frame polymer, and
(c) depositing the at least one layer of polymer fibers (4) on the frame (1), optionally by electrospinning,
optionally further comprising a subsequent step of:
(d) permanently adhering the fibers (4) to the frame.

14. The process of claim 13, further comprising before step (b) a step
(a) generating at least one layer of polymer fibers (4) comprising an organic fiber polymer by electrospinning,
wherein in step (c) the layer of polymer fibers (4) is deposited on the frame (1) by moving the frame (1) through the layer of fibers (4) generated by electrospinning.

15. The wound dressing obtained or obtainable by the process of any of claims 13 or 14.

## Patentansprüche

1. Wundverband, umfassend einen Rahmen (1), der mindestens ein organisches Rahmenpolymer und mindestens eine Schicht von Fasern (4) umfasst, die den Rahmen (1) überspannt, wobei die Fasern (4) mindestens ein organisches Faserpolymer umfassen.

2. Wundverband nach Anspruch 1, wobei die Fasern (4) in der mindestens einen Schicht im Wesentlichen parallel ausgerichtet sind.

3. Wundverband nach einem der vorhergehenden Ansprüche, wobei die Fasern durch Elektrospinnen erhalten wurden.

4. Wundverband nach einem der vorhergehenden Ansprüche, der mehrere Schichten von Fasern (4) umfasst, die den Rahmen überspannen, wobei die Fasern in verschiedenen Richtungen in verschiedenen Schichten im Wesentlichen parallel ausgerichtet sind.

5. Wundverband nach einem der vorhergehenden Ansprüche, wobei
das Rahmenpolymer und/oder das Faserpolymer ein biologisch abbaubares Polymer ist; und/oder
das Rahmenpolymer und/oder das Faserpolymer ausgewählt ist aus der Gruppe bestehend aus Polyester, Polyether, Polyaminosäure, Polyacrylat, Acrylamidpolymeren, Acrylatpolymeren, Silicon, Polypeptid, Polysaccharid und/oder Polyolefin, wobei vorzugsweise das Rahmenpolymer und/oder das Faserpolymer ausgewählt ist aus Elastin, Kollagen, Polyurethan, ε-Polycaprolacton (PCL), Polylactid und/oder Polyglycolsäure; und/oder
das Rahmenpolymer und das Faserpolymer die gleichen Polymere sind.

6. Wundverband nach einem der vorhergehenden Ansprüche, wobei die mindestens eine Schicht von Fasern (4) porös ist.

7. Wundverband nach einem der vorhergehenden Ansprüche, der ein Hydrogel umfasst, vorzugsweise ein Hydrogel, das mindestens einen Wirkstoff umfasst.

8. Wundverband nach einem der vorhergehenden Ansprüche, wobei der Rahmen (1) aus einem Polymerfilm oder einem Vliesfasermaterial besteht, vorzugsweise in der Form eines Rings.

9. Wundverband nach einem der vorhergehenden Ansprüche, wobei die Gestalt des Wundverbandes konvex ist, vorzugsweise ausgestaltet, um eine durchschnittliche Hornhaut abzudecken.

10. Wundverband nach einem der vorhergehenden Ansprüche, wobei die mindestens eine Schicht von Fasern, die den Rahmen überspannt, dauerhaft an dem Rahmen haftet.

11. Wundverband nach einem der vorhergehenden Ansprüche, wobei die Schicht oder Schichten von Fasern (4), die den Rahmen (1) überspannen, in das Material eingebettet sind, das den Rahmen (1) bildet.

12. Wundverband nach einem der vorhergehenden Ansprüche, wobei der Wundverband zur Augenbehandlung, vorzugsweise zur Behandlung der Hornhaut, und/oder zur Augenoperation, vorzugsweise zur Operation der Hornhaut, bestimmt ist.

13. Verfahren zur Herstellung eines Wundverbandes der vorhergehenden Ansprüche, umfassend die Schritte
(b) Bereitstellen des Rahmens (1), der das organische Rahmenpolymer umfasst, und
(c) Abscheiden der mindestens einen Schicht von Polymerfasern (4) auf dem Rahmen (1), gegebenenfalls durch Elektrospinnen,
gegebenenfalls weiter umfassend einen nachfolgenden Schritt:
(d) dauerhaftes Anhaften der Fasern (4) an dem Rahmen.

14. Verfahren nach Anspruch 13, vor dem Schritt (b) ferner umfassend einen Schritt
(a) Erzeugen mindestens einer Schicht von Polymerfasern (4), die ein organisches Faserpolymer umfassen, durch Elektrospinnen,
wobei in Schritt (c) die Schicht von Polymerfasern (4) durch Bewegen des Rahmens (1) durch die durch Elektrospinnen erzeugte Schicht von Fasern (4) auf dem Rahmen (1) abgeschieden wird.

15. Wundverband, erhalten oder erhältlich durch das Verfahren nach einem der Ansprüche 13 oder 14.

## Revendications

1. Pansement pour plaie, comprenant un cadre (1), qui comprend au moins un polymère de cadre organique et au moins une couche de fibres (4) chevauchant le cadre (1), les fibres (4) comprenant au moins un polymère de fibre organique.

2. Pansement pour plaie selon la revendication 1, les fibres (4) dans l'au moins une couche étant alignées de manière sensiblement parallèle.

3. Pansement pour plaie selon l'une quelconque des revendications précédentes, les fibres ayant été obtenues par électrofilage.

4. Pansement pour plaie selon l'une quelconque des revendications précédentes, qui comprend plusieurs couches de fibres (4) chevauchant le cadre, les fibres étant alignées de manière sensiblement parallèle dans différentes directions dans différentes couches.

5. Pansement pour plaie selon l'une quelconque des revendications précédentes,
le polymère de cadre et/ou le polymère de fibre étant un polymère biodégradable ; et/ou
le polymère de cadre et/ou le polymère de fibre étant choisi dans le groupe constitué par un polyester, un polyéther, un poly(acide aminé), un polyacrylate, des polymères d'acrylamide, des polymères d'acrylate, une silicone, un polypeptide, un polysaccharide et/ou une polyoléfine, préférablement le polymère de cadre et/ou le polymère de fibre étant choisi(s) parmi l'élastine, le collagène, un polyuréthane, une ε-polycaprolactone (PCL), un polylactide, et/ou un poly(acide glycolique) ; et/ou
le polymère de cadre et/ou le polymère de fibre étant les mêmes polymères.

6. Pansement pour plaie selon l'une quelconque des revendications précédentes, l'au moins une couche de fibres (4) étant poreuse.

7. Pansement pour plaie selon l'une quelconque des revendications précédentes, qui comprend un hydrogel, préférablement un hydrogel comprenant au moins un agent actif.

8. Pansement pour plaie selon l'une quelconque des revendications précédentes, le cadre (1) étant constitué d'un film de polymère ou d'un matériau fibreux non-tissé, préférablement sous la forme d'un anneau.

9. Pansement pour plaie selon l'une quelconque des revendications précédentes, la forme du pansement pour plaie étant convexe, préférablement conçue pour couvrir une cornée moyenne.

10. Pansement pour plaie selon l'une quelconque des revendications précédentes, l'au moins une couche de fibres chevauchant le cadre adhérant de manière permanente au cadre.

11. Pansement pour plaie selon l'une quelconque des revendications précédentes, la couche ou les couches de fibres (4) chevauchant le cadre (1) étant incorporée (s) dans le matériau constituant le cadre (1).

12. Pansement pour plaie selon l'une quelconque des revendications précédentes, le pansement pour plaie étant pour un traitement de l'œil, préférablement pour le traitement de la cornée, et/ou pour une chirurgie de l'œil, préférablement pour une chirurgie de la cornée.

13. Procédé pour la fabrication d'un pansement pour plaie selon les revendications précédentes, comprenant les étapes de
(b) mise à disposition du cadre (1) qui comprend le polymère de cadre organique, et
(c) dépôt de l'au moins une couche de fibres (4) de polymère sur le cadre (1), éventuellement par électrofilage,
éventuellement comprenant en outre une étape subséquente de :
(d) adhérence de manière permanente des fibres (4) au cadre.

14. Procédé selon la revendication 13, comprenant en outre avant l'étape (b) une étape de
(a) génération d'au moins une couche de fibres (4) de polymère comprenant un polymère de fibre organique par électrofilage,
dans lequel dans l'étape (c) la couche de fibres (4) de polymère est déposée sur le cadre (1) par déplacement du cadre (1) à travers la couche de fibres (4) générée par électrofilage.

15. Pansement pour plaie obtenu ou pouvant être obtenu par le procédé selon l'une quelconque des revendications 13 et 14.
